# EUROPEAN PATENT SPECIFICATION

(11) **EP 4 090 284 B1**
(45) Date of publication and mention of the grant of the patent: **16.07.2025**
(21) Application number: 21702572.5
(22) Date of filing: 15.01.2021
(51) Int. Cl.: A61F 2/12, A61L 27/38, A61L 27/52

(54) **HYDROGEL-BASED IMPLANTABLE DEVICE**
IMPLANTIERBARE VORRICHTUNG AUF HYDROGELBASIS
DISPOSITIF IMPLANTABLE À BASE D'HYDROGEL

(30) Priority: 16.01.2020 US 202016744810
(43) Date of publication of application: 23.11.2022
(73) Proprietor: Hologic, Inc., Marlborough, MA 01752 (US)
(72) Inventor: NIREMBERG, Danielle, Marlborough, MA 01752 (US)
(74) Representative: Hoyng Rokh Monegier B.V.
(86) International application number: PCT/US2021/013621
(87) International publication number: WO 2021/146549

(56) References cited:
- WO-A1-2015/002707
- WO-A1-2019/217765
- AU-B2- 2015 301 432
- US-A1- 2007 104 695
- US-A1- 2012 040 461
- JEANNINE COBURN ET AL: "Biomimetics of the extracellular matrix: an integrated three-dimensional fiber-hydrogel composite for cartilage tissue engineering", SMART STRUCTURES AND SYSTEMS, vol. 7, no. 3, 25 March 2011 (2011-03-25), pages 213 - 222, XP055172968, ISSN: 1738-1584, DOI: 10.12989/sss.2011.7.3.213

## Description

### BACKGROUND

If a suspicious area within the breast is discovered through screening procedures, a breast biopsy may be performed to remove a sample of breast tissue from the suspicious area for diagnostic evaluation. If based on the diagnostic evaluation the suspicious area is determined to be abnormal breast tissue, the abnormal breast tissue and at least some portions of the surrounding breast tissue may be resected surgically. The removal of the breast tissue may create a cavity at a site of the resection.

It is with respect to these and other general considerations that the aspects disclosed herein have been made. Also, although relatively specific problems may be discussed, it should be understood that the examples should not be limited to solving the specific problems identified in the background or elsewhere in this disclosure.

AU2015301432 describes composite materials comprising a hydrogel and a nanostructure for use in methods for reconstruction of soft tissue. US2007/104695 describes breast templates for delivering stem cell formulations to a breast defect of a patient. WO2019/217765 describes nanofiber-hydrogel composites for cell and tissue delivery and methods for manufacturing.

### SUMMARY

Examples of the present disclosure describe devices and methods for facilitating post-resection tissue formation to accelerate healing. In an aspect, the technology relates to a method to accelerate healing after a resection of tissue. The method includes preparing a hydrogel scaffold with encapsulated cells corresponding to a type of the tissue resected, and integrating the hydrogel scaffold with a frame to form an implantable device for insertion into a cavity created by the resection.

In an example, a hydrogel precursor solution is prepared, and gelation of the hydrogel precursor solution initiated to form the hydrogel scaffold. In another example, preparing the hydrogel scaffold with the encapsulated cells includes removing a sample of tissue during a biopsy, where the sample of tissue may be a same type of tissue as the tissue resected, isolating cells from the sample of tissue, harvesting and expanding the cells in vitro, mixing the cells in the hydrogel precursor solution to form a precursor-cell solution, and initiating gelation of the precursor-cell solution to form the hydrogel scaffold with the encapsulated cells. In a further example, the sample of tissue is removed during the biopsy of a patient undergoing the resection such that the cells encapsulated in the hydrogel scaffold are cells of the patient, where the sample of tissue removed comprise at least in part a healthy tissue.

In another example, integrating the hydrogel scaffold with the frame includes at least partially surrounding one or more portions of the frame with the hydrogel scaffold. A hydrogel precursor solution is applied to the one or more portions of the frame, and gelation of the hydrogel precursor solution initiated to form the hydrogel scaffold that at least partially surrounds the one or more portions of the frame. In an example, the frame is encapsulated in the hydrogel scaffold by submerging the frame in the hydrogel precursor solution prior to the gelation. In another example, the hydrogel precursor solution is applied to an at least partially open structure of the frame such that the hydrogel scaffold is formed within the frame upon gelation. In a further example, an application of the hydrogel precursor solution to the one or more portions of the frame is determined based on growth patterns of new tissue formed in the cavity. In a yet further example, at least one of a size and a shape of the frame may be selected based at least in part on at least one of a size and a shape of the cavity.

In another aspect, the technology relates to an implantable device to accelerate healing after a resection of tissue. The implantable device includes a hydrogel scaffold including encapsulated cells that correspond to a type of the tissue resected to facilitate tissue formation within a cavity formed by the resection upon insertion of the implantable device into the cavity, and a frame comprising one or more portions that are at least partially surrounded by the hydrogel scaffold.

In an example, the frame is encapsulated in the hydrogel scaffold. In another example, the frame has an at least partially open structure, and the hydrogel scaffold may be formed within the frame. In a further example, the hydrogel scaffold and the frame are bioabsorbable. In a yet further example, the frame includes one or more markers positioned along the one or more portions of the frame, and the one or more markers may be comprised of a non-bioabsorbable, radiopaque material. In an example, at least one of a size and a shape of the frame may be selected based at least in part on at least one of a size and a shape of the cavity formed by the resection.

In a further aspect, the technology relates to a method to accelerate healing after a resection of tissue. The method includes preparing a hydrogel scaffold with encapsulated cells corresponding to a type of the tissue resected, integrating the hydrogel scaffold with a frame to form an implantable device, where one or more portions of the frame are at least partially surrounded by the hydrogel scaffold, and inserting the implantable device into a cavity formed by the resection.

In an example, preparing the hydrogel scaffold with the encapsulated cells includes removing a sample of tissue during a biopsy, where the sample of tissue is a same type of tissue as the tissue resected, isolating cells from the sample of tissue, harvesting and expanding the cells in vitro, mixing the cells in a hydrogel precursor solution to form a precursor-cell solution, and initiating gelation of the precursor-cell solution to form the hydrogel scaffold with the encapsulated cells. In another example, the precursor-cell solution is applied to the one or more portions of the frame, and gelation of the precursor-cell solution is initiated to form the hydrogel scaffold that at least partially surrounds the one or more portions of the frame.

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter. Additional aspects, features, and/or advantages of examples will be set forth in part in the description which follows and, in part, will be apparent from the description, or may be learned by practice of the disclosure.

The present invention relates to a method for preparing an implantable device and to an implantable device as set forth in the appended claims. The methods of using the implantable device and to accelerate healing after resection of tissue do not form part of the invention.

### BRIEF DESCRIPTION OF THE DRAWINGS

Non-limiting and non-exhaustive examples are described with reference to the following figures.
FIG. 1 depicts an example configuration of an implantable device for insertion into a resection cavity.
FIG. 2 depicts another example configuration of an implantable device for insertion into a resection cavity.
FIG. 3 depicts a further example configuration of an implantable device for insertion into a resection cavity.
FIG. 4 depicts an example hydrogel scaffold with encapsulated cells.
FIG. 5 depicts an example process for preparing a hydrogel scaffold with encapsulated cells.
FIG. 6 depicts an example frame integrated with a hydrogel scaffold to form an implantable device.
FIG. 7 depicts example frame configurations.
FIG. 8 depicts a method to accelerate healing after a resection of tissue.

### DETAILED DESCRIPTION

Based on results of a diagnostic evaluation, a resection surgery may be performed to remove abnormal breast tissue. The removal of the breast tissue may create a cavity at a site of the resection. Over time, new breast tissue may form in the cavity. However, if the cavity is left void while the new breast tissue forms, deformities to the breast may occur that affect the look and/or feel of the breast. Additionally, identification of the site of resection may be important for subsequent procedures and/or imaging, among other examples. Therefore, implantable surgical markers may be inserted into the cavity during the surgery that provide temporary structural support within the cavity as the new breast tissue forms, as well as enable identification of the site for a prolonged period of time post-resection. However, conventional markers fail to facilitate new breast tissue formation within the cavity to accelerate healing post-resection. A bioreactor that induces cell in tissue integration may be one example solution.

Examples as described herein provide a hydrogel-based implantable device for insertion within the cavity. The implantable device may include a hydrogel scaffold with encapsulated cells serving as an example bioreactor to induce cell in tissue integration. The encapsulated cells may correspond to a type of the tissue resected, and may be cells of a patient undergoing the resection. For example, a sample of tissue corresponding to a same type of tissue resected may be removed during a biopsy performed on the patient, where healthy, tissue-specific cells may be isolated from the sample of tissue and harvested in vitro for encapsulation in the hydrogel scaffold. The encapsulated cells may interact with native cells of the breast to facilitate new tissue formation within the hydrogel scaffold and other areas of the cavity upon insertion of the implantable device into the cavity.

The implantable device includes a frame having one or more portions at least partially surrounded by the hydrogel scaffold. The frame may be bioabsorbable providing temporary structural support within the cavity during the tissue formation facilitated by the encapsulated cells of the hydrogel scaffold. Additionally, a plurality of markers comprised of non-bioabsorbable, radiopaque material may be positioned along the frame to enable identification of the site of resection.

For clarity, an implantable device to facilitate tissue reformation and accelerate healing within the breast are described herein. However, a similar implantable device may be inserted within resection cavities formed from the removal of tissues other than breast tissue to facilitate tissue reformation and accelerate healing post-resection.

In describing examples illustrated in the drawings, specific terminology is employed for the sake of clarity. However, the disclosure of this patent specification is not intended to be limited to the specific terminology so selected and it is to be understood that each specific element includes all technical equivalents that operate in a similar manner.

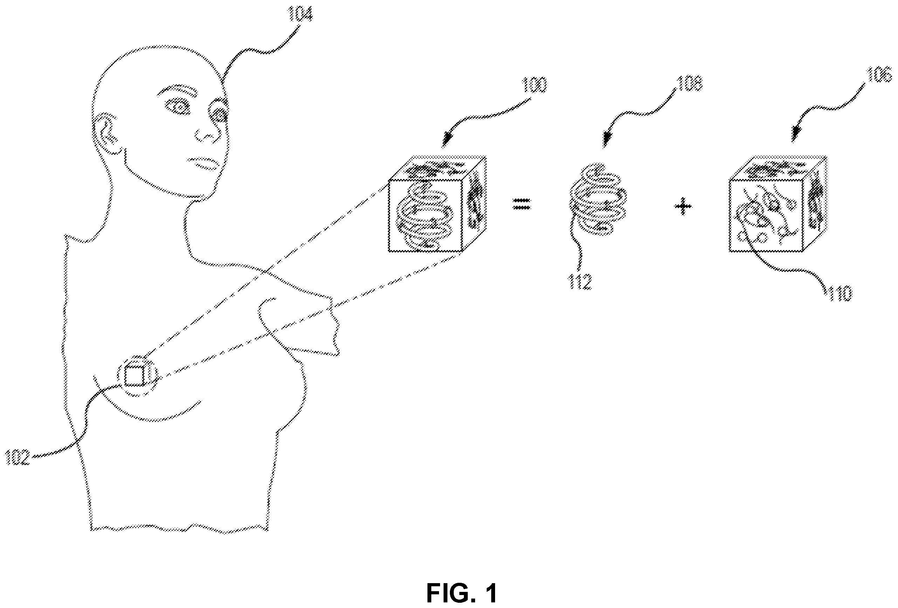

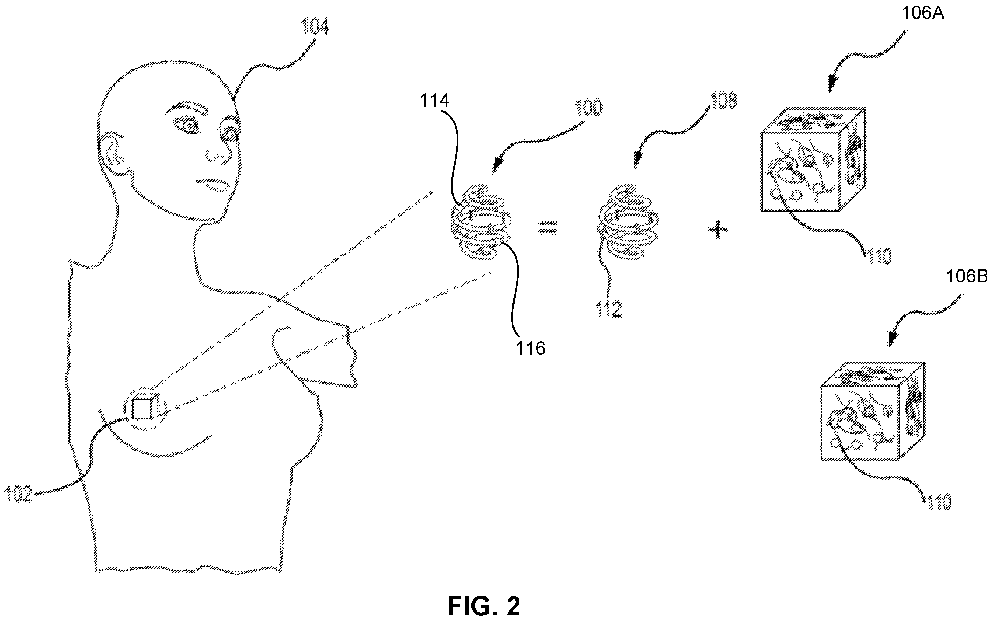

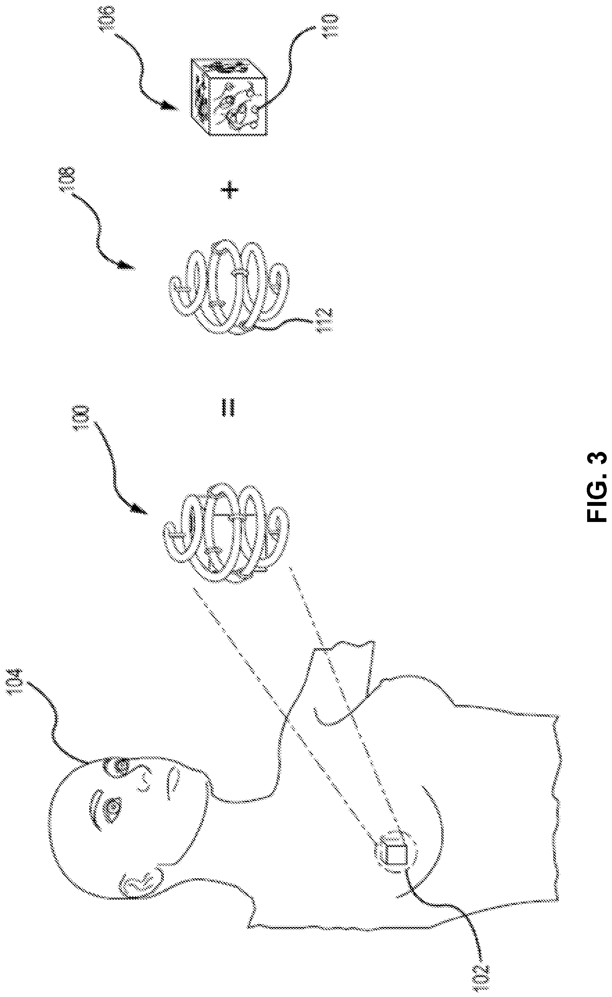

FIG. 1 depicts an example configuration of an implantable device 100 for insertion into a resection cavity 102. FIG. 2 depicts another example configuration of the implantable device 100. FIG. 3 depicts a further example configuration of the implantable device 100. Referring concurrently to FIGS. 1, 2, and 3, a surgical resection of breast tissue may be performed on a patient 104. In one example, a portion of breast tissue may be determined through a diagnostic evaluation to be abnormal (e.g., comprise malignant cells), and the patient 104 may have a local wide excision or lumpectomy performed to remove the abnormal breast tissue, as well as at least a small portion of normal breast tissue surrounding the abnormal breast tissue. The removal of the breast tissue creates the cavity 102 at a site of the removal (e.g., at a site of resection). If the cavity 102 is left void while new breast tissue forms within the cavity 102, deformities to the breast may occur that affect the look and/or feel of the breast. Undergoing the surgical resection and any other necessary post-resection procedures, such as a radiation or chemotherapy, may understandably be a traumatic experience for the patient 104, which may further be exacerbated by such deformities.

The implantable device 100 may be inserted into the cavity 102 at the site of resection immediately following the removal of the breast tissue as part of the surgical procedure. The implantable device 100 may include a hydrogel scaffold 106 and a frame 108. The hydrogel scaffold 106 may facilitate formation of new breast tissue within the cavity 102 to accelerate the post-resection healing process, while the frame 108 may provide structural support within the cavity 102 to reduce deformities and enable identification of the site of resection during any post-resection procedures.

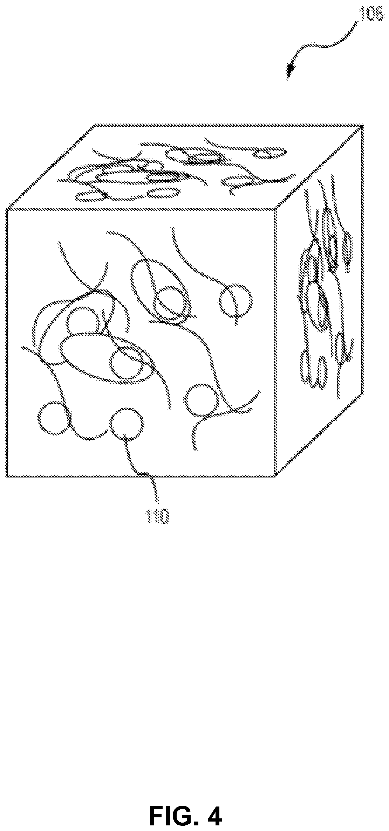

As described in greater detail with respect to FIG. 4, the hydrogel scaffold 106 may comprise a three-dimensional (3D) network of cross-linked, hydrophilic polymer chains that mimics a microenvironment of the breast, supporting cell growth. Therefore, cells 110 may be encapsulated in the hydrogel scaffold 106 to proliferate and differentiate. In some examples, the cells 110 may be healthy cells of the patient 104 undergoing the resection. For example, the cells 110 may be obtained from healthy breast tissue of the patient 104 removed during a biopsy performed for diagnostic evaluation of the breast tissue (e.g., to determine whether a resection is necessary). The hydrogel scaffold 106 may include growth factors to stimulate growth of the cells 110, among other functions, and may provide one or more of chemical and mechanical cues for directing behavior of the cells 110 in order to mirror how the cells 110 would behave in the microenvironment present in the breast tissue. For example, chemical cues may indicate a site for cell adhesion and mechanical cues may direct differentiation of the cells. The chemical and/or mechanical cues provided may be based on a type of polymer material from which the hydrogel scaffold 106 is formed. Additionally, the hydrogel scaffold 106 may be customized to more closely resemble a density of the patient's breast tissue by selecting a particular type of polymer material to form the hydrogel scaffold 106.

While some cell growth is ideal within the hydrogel scaffold 106, it is preferable that tissue formation has not yet occurred before insertion of the implantable device 100 into the cavity 102 such that the cells 110 interact with native cells of the breast to form the new breast tissue. For example, upon insertion of the implantable device 100 into the cavity 102, native cells may migrate into the hydrogel scaffold 106 to form new breast tissue along with some of the encapsulated cells 110 within the hydrogel scaffold 106. Additionally, some of the encapsulated cells 110 may migrate out of the hydrogel scaffold 106 to form new breast tissue along with native breast cells in other areas within the cavity 102. The interaction between the encapsulated cells 110 and the native cells may increase a rate at which new breast tissue is formed, thereby accelerating the healing process.

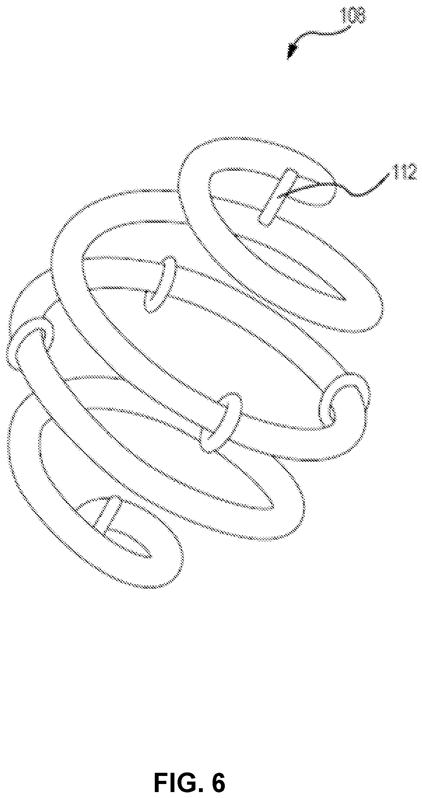

The frame 108 may be an open structure, such as a helix as illustrated in FIGS. 1, 2, and 3. In other examples, the frame may be a partially open structure or a closed structure. As described in greater detail with respect to FIG. 6, the frame 108 may be bioabsorbable, providing temporary structural support within the cavity 102 to reduce breast deformities created while tissue formation within the cavity 102 is being facilitated by the cells 110 of the hydrogel scaffold 106. Additionally, the frame 108 may include a plurality of markers 112 spaced along one or more portions of the frame 108. The markers 112 may be comprised of a non-bioabsorbable, radiopaque material such that the markers 112 remain at the site of resection for a prolonged period of time post-resection to enable identification of the site via imaging processes.

The hydrogel scaffold 106 may be integrated with the frame 108 to form the implantable device 100. In some examples, more than one hydrogel scaffold 106 may be integrated with the frame 108 to form the implantable device 100, as illustrated in FIG. 2. To integrate the hydrogel scaffold 106 and the frame 108, one or more portions of the frame 108 may be at least partially surrounded by the hydrogel scaffold 106. As described in greater detail with respect to FIG. 4, formation of the hydrogel scaffold 106 may involve preparation of a liquid precursor solution, also referred to herein as a hydrogel precursor solution, that is transitioned into a semi-solid material using physical crosslinking or chemical crosslinking (e.g., via gelation). The cells 110 may be mixed into the hydrogel precursor solution prior to gelation. The hydrogel precursor solution may be applied to one or more portions of the frame 108, and gelation may be initiated to form the hydrogel scaffold 106. In some examples, gelation may be initiated using photopolymerization (e.g., by placing the hydrogel precursor solution under ultraviolet light). In other examples, gelation may be initiated by changes in pH or temperature, or ion addition, among other similar methods known to a person having ordinary skill in the art. Upon gelation one or more portions of the frame 108 may be at least partially surrounded by the hydrogel scaffold 106.

In some examples, to apply the hydrogel precursor solution, the frame 108 may be placed or positioned in the hydrogel precursor solution that is contained within a receptacle, such as a petri dish or well plate, among other similar receptacles. As one example, the frame 108 may be submerged in the hydrogel precursor solution such that each portion of the frame 108 may be entirely surrounded by the hydrogel scaffold 106 upon gelation, as illustrated in FIG. 1. In other words, the frame 108 may be encapsulated in the hydrogel scaffold 106. Encapsulation of the frame 108 in the hydrogel scaffold 106 may allow the breast to feel more natural to the patient 104 post-resection. For example, the gel-like feel of the hydrogel scaffold 106 may more closely resemble the feel of the breast than the harder feel of the frame 108. Additionally, the encapsulation of the frame 108 in the hydrogel scaffold 106 may help reduce chances of rejection of the implantable device 100 by the patient's body due to the biocompatibility of the hydrogel scaffold 106 with the breast environment. As another example, the frame 108 may be placed or positioned in the hydrogel precursor solution such that the frame 108 is not entirely submerged and only some portions of the frame 108 are exposed to the hydrogel precursor solution. Upon gelation, only those portions of the frame 108 exposed to the hydrogel precursor solution are at least partially surrounded with the hydrogel scaffold 106.

In other examples, where more than one hydrogel scaffold 106 may be integrated with the frame 108 to form the implantable device 100, the above described application of hydrogel precursor solution to one or more portions of the frame 108 and subsequent gelation may be repeated for each hydrogel scaffold 106. For example, as illustrated in FIG. 2, at least two hydrogel scaffolds 106 (e.g., first hydrogel scaffold 106A and second hydrogel scaffold 106B) are formed at a different portion or segment of the frame 108. In some examples, each hydrogel scaffold 106 is formed such that it surrounds a perimeter of the portion or segment (e.g., forms a hydrogel ring around the portion or segment). For example, a first portion or segment 114 of the frame 108 may be positioned into the hydrogel precursor solution so that the hydrogel precursor solution surrounds only that first portion or segment 114, and then gelation may be initiated to form the first hydrogel scaffold 106A surrounding the first portion or segment 114. Subsequently, a second portion or segment 116 of the frame 108 may be positioned into the hydrogel precursor solution so that the hydrogel precursor solution surrounds only that second portion or segment 116, and then gelation may be initiated to form the second hydrogel scaffold 106B surrounding the second portion or segment 116. Additional hydrogel scaffolds 106 may be formed to surround additional portions or segments of the frame 108 in a same or similar manner.

In further examples, the frame 108 may have at least a partially open structure, and the hydrogel precursor solution may be applied to the open structure to form the hydrogel scaffold 106 within the frame 108. For example, based on a structure of the frame 108, the frame 108 may serve as a mold or be positioned within a mold having a similar shape to the frame 108. As one example, the frame 108 may serve as a mold if the frame 108 has a partially open structure such as a hollow cone. As another example, the frame 108 may be positioned within the mold if the frame 108 has an open structure such as a helix. The hydrogel precursor solution may be poured into the open structure, and gelation may be initiated to form the hydrogel scaffold 106. Upon gelation, the hydrogel scaffold 106 may be formed within the frame 108, as illustrated in FIG. 3. In one example, there may be an additional support structure within the frame 108 around which the hydrogel scaffold 106 is formed upon gelation.

In some examples, the integration of the hydrogel scaffold 106 and the frame 108 may be based on growth patterns of new tissue formed in the cavity 102 (e.g., based on where growth of breast tissue initially occurs within the cavity 102). For example, if breast tissue naturally grows first in areas surrounding the frame 108 within the cavity 102, a frame 108 having an open structure may be selected for integration and the hydrogel scaffold 106 may be formed within the frame 108, as illustrated in FIG. 3. This may promote the migration of native cells into the hydrogel scaffold 106 to help facilitate breast tissue growth within the frame 108.

FIG. 4 depicts an example hydrogel scaffold 106 with encapsulated cells 110. The hydrogel scaffold 106 comprises a bioabsorbable, 3D network of hydrophilic polymer chains held together by cross-links. Formation of the hydrogel scaffold 106 may involve preparation of a hydrogel precursor solution comprised of at least one or more hydrophilic polymers that is transitioned into a semi-solid material using physical crosslinking or chemical crosslinking (e.g. via gelation). In some examples, the hydrogel scaffold 106 may be formed from one or more synthetic polymer materials, including one or more of poly(2-hydroxyethyl methacrylate) (pHEMA), polyvinylpyrrolidone (PVP), polyethylene glycol diacrylate (PEGDA) and poly(vinyl alcohol) (PVA), among other similar polymers. In other examples, the hydrogel scaffold 106 may be formed from one or more natural polymer materials such as collagen, fibrin, and alginate, among other natural polymer materials. In further examples, the hydrogel scaffold 106 may be formed from hydrophilic polymer materials that are a hybrid of natural and synthetic polymer materials. These polymer materials are provided merely as examples, and those having skill in the art will recognize and understand additional or different polymer materials that may be used to form a hydrogel scaffold, such as the hydrogel scaffold 106.

A type of crosslinking performed may be based on the type of polymer materials selected. Additionally, depending on the type of polymer material selected and the crosslinking to be performed, additional chemical modifications may be made to the hydrogel precursor solution, such as an addition of cross-linking agents and/or initiators. In some examples, the polymer materials may be selected based on factors associated with an anatomy of the breast of the patient 104. For example, if the breast of the patient 104 is dense, particular types of polymer materials may be selected to more closely resemble the dense breast tissue.

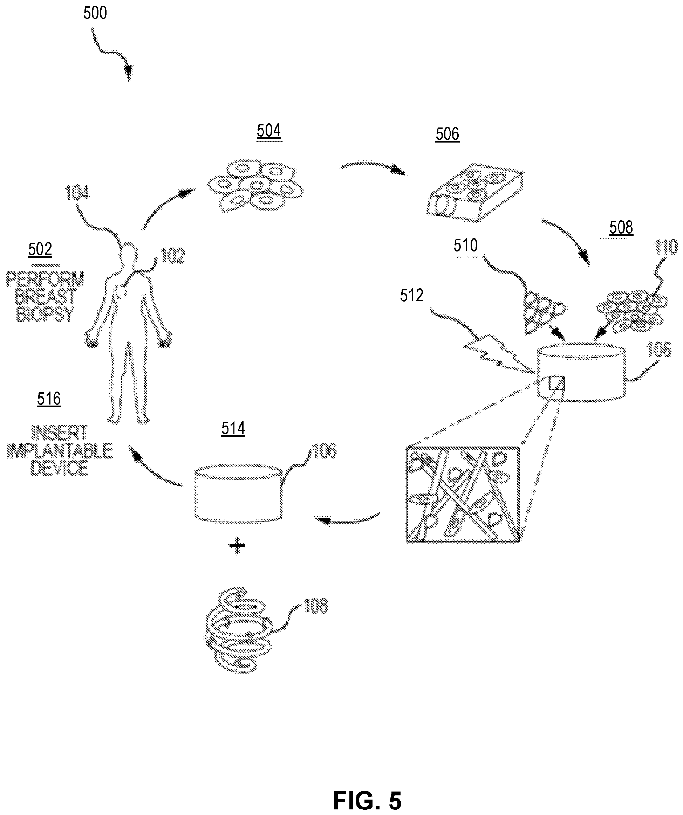

The hydrogel scaffold 106 may mimic a microenvironment of the breast, supporting cell growth. Therefore, cells 110 may be encapsulated in the hydrogel scaffold 106 to proliferate and differentiate. In some examples, the cells 110 may be healthy cells of the patient 104 undergoing the resection. For example, the cells 110 may be obtained from healthy breast tissue of the patient 104 removed during a biopsy performed for diagnostic evaluation of the breast tissue (e.g., to determine whether the resection was necessary). A method for isolating and encapsulating the cells 110 in the hydrogel scaffold 106 is described in greater detail in FIG. 5. Additionally, the hydrogel scaffold 106 may include growth factors to stimulate growth of the cells 110, among other functions, and provide one or more of chemical and mechanical cues for directing behavior of the cells 110 in order to mirror how the cells 110 would behave in the microenvironment present in the breast tissue. For example, chemical cues may indicate a site for cell adhesion and mechanical cues may direct differentiation of the cells. The chemical and/or mechanical cues provided may be based on the type(s) of polymer materials from which the hydrogel scaffold 106 is formed.

While some cell growth is ideal within the hydrogel scaffold 106, it is preferable that tissue formation has not yet occurred before insertion of the implantable device 100 into the cavity 102 such that the cells 110 interact with native cells to form the new breast tissue. For example, once the implantable device 100 is inserted into the cavity 102, some of the cells 110 may migrate out of the hydrogel scaffold 106 to other areas of the cavity 102, and native cells may migrate into the hydrogel scaffold 106 to engage in intercellular communication. The migration occurring in and out of the hydrogel scaffold 106 may promote and accelerate breast tissue formation within the hydrogel scaffold 106 as well as other areas within the cavity 102. The acceleration of the breast tissue formation may correspondingly accelerate the post-resection healing process. Over time, the hydrogel scaffold 106 may be absorbed by the patient's body.

FIG. 5 depicts an example process 500 for preparing a hydrogel scaffold with encapsulated cells. If a suspicious area within the breast is discovered through screening procedures, for example, a breast biopsy may be performed to remove a sample of breast tissue from the suspicious area for diagnostic evaluation at operation 502. The sample of breast tissue removed during the breast biopsy may include the suspicious area, as well as portions of healthy breast tissue surrounding the suspicious area.

If a resection is determined to be necessary based on the diagnostic evaluation, the cells 110 to be encapsulated in the hydrogel scaffold 106 may be obtained from the portions of healthy breast tissue from the breast biopsy sample. For example, at operation 504, tissue-specific cells may be isolated from the portions of healthy breast tissue. At operation 506, the cells may be harvested and expanded in vitro to increase a number of cells to be encapsulated in the hydrogel scaffold 106 (e.g., encapsulated cells 110).

At operation 508, the hydrogel scaffold 106 may be prepared. For example, a hydrogel precursor solution may be prepared, and the hydrogel precursor solution may undergo gelation to form the hydrogel scaffold 106. The hydrogel precursor solution may be comprised of one or more hydrophilic polymers, where the polymers may be natural, synthetic, or a hybrid of natural and synthetic. In some examples, the hydrogel scaffold 106 may be formed from one or more of pHEMA, PVP, PEGDA, and PVA, among other similar polymers. During gelation, the polymers may undergo physical or chemical crosslinking to form a 3D network of cross-linked, polymer chains that mimics a microenvironment of the breast, supporting cell growth. A type of crosslinking performed may be based on the type of polymers used to prepare the hydrogel precursor solution. Additionally, depending on the type of polymer selected and the crosslinking to be performed, additional chemical modifications may be made to the hydrogel precursor solution, such as the addition of cross-linking agents and/or initiators.

Prior to gelation, the cells 110 may be mixed into the hydrogel precursor solution (e.g., forming a precursor-cell solution) to enable even distribution of the cells 110 throughout an entirety of the hydrogel scaffold 106 upon gelation. Additionally, growth factors 510 may be incorporated in the precursor solution that stimulate growth of the cells 110, among other functions. In some examples, the hydrogel precursor solution may also include a buffer solution to control a pH level of the hydrogel precursor solution.

Additionally, the hydrogel scaffold 106 may provide one or more of chemical and mechanical cues 512 for directing behavior of the cells 110 in order to mirror how the cells 110 would behave in the microenvironment present in the breast tissue. For example, chemical cues may indicate a site for cell adhesion and mechanical cues may direct differentiation of the cells 110. The chemical and/or mechanical cues 512 provided may be based on the type(s) of polymer from which the hydrogel scaffold 106 is formed.

At operation 514, the hydrogel scaffold 106 may be integrated with the frame 108 to form the implantable device 100. The integration may occur prior to the gelation of the precursor-cell solution. In some examples, the precursor-cell solution may be placed within a receptacle, such as a petri dish or a well plate, among other similar receptacles. The frame 108 may then be positioned in the receptacle such that one or more portions of the frame 108 are at least partially exposed to the precursor-cell solution. Gelation may be initiated causing the hydrogel scaffold to at least partially surround the one or more portions of the frame 108. In some examples, gelation may be initiated using photopolymerization (e.g., placing the hydrogel precursor solution under ultraviolet light). In other examples, gelation may be initiated by changes in pH or temperature, or ion addition, among other similar methods known to a person having ordinary skill in the art.

In one example, the frame 108 may be submerged within the precursor-cell solution such that the frame 108 is encapsulated in the hydrogel scaffold 106 upon gelation. In other examples, the frame 108 may have an at least partially open structure. The precursor-cell solution may be applied to (e.g., poured into) the open structure, and gelation may be initiated such that the hydrogel scaffold 106 is formed within the frame 108.

Once the hydrogel scaffold 106 and the frame 108 have been integrated to form the implantable device, the implantable device 100 may then be inserted into the cavity 102 at operation 516. The implantable device 100 may be inserted immediately following the removal of breast tissue that created the cavity 102.

FIG. 6 depicts an example frame 108 integrated with a hydrogel scaffold 106 to form an implantable device 100. The frame 108 may have an open structure, as illustrated in FIG. 6. In other examples, the frame 108 may be a partially open structure or a closed structure. In some examples, for any structure type, one or more portions of the frame 108 may be placed or positioned in a hydrogel precursor solution that is contained within a receptacle, and gelation may be initiated to form the hydrogel scaffold 106 such that the one or more portions of the frame 108 are at least partially surrounded by the hydrogel scaffold 106. In other examples, the frame 108 may serve as a mold (e.g., if the frame 108 is a partially open structure such as a hollow cone) or be positioned within a mold having a similar shape to the frame (e.g., if the frame 108 is an open structure such as a helix as illustrated in FIG. 6). The hydrogel precursor solution may be poured into the open structure, and gelation may be initiated to form the hydrogel scaffold 106 within the frame 108.

The frame 108 may be formed from a bioabsorbable material and provide temporary structural support within the cavity 102. Due to the bioabsorbable nature of the hydrogel scaffold 106, the hydrogel scaffold 106 at least partially surrounding the one or more portions of the frame 108 does not interfere with the absorption of the frame 108 by the body, even when the frame 108 is encapsulated in the hydrogel scaffold 106. A rate at which the frame 108 absorbs may be based on a type of the bioabsorbable material. In some examples, the bioabsorbable material may be selected such that the frame 108 is absorbed at a faster or slower rate than the hydrogel scaffold 106. In other examples, the bioabsorbable material may be selected such that the frame 108 is absorbed at a same rate as the hydrogel scaffold 106.

A size and shape of the frame 108, as well as structure, may be selected based at least in part on a size and shape of the cavity 102. For example, a size of frame 108 may be slightly smaller than the size of the cavity 102 so that the implantable device 100 may fit within the cavity, but may otherwise be similar in size and shape of the cavity 102 to provide adequate structural support.

In some examples, the frame 108 may include a plurality of markers 112 spaced along one or more portions of the frame 108. The markers 112 may be comprised of a non-bioabsorbable, radiopaque material such that the markers 112 remain at the site of resection for a prolonged period of time post-resection to enable identification of the site via imaging processes. In one example, the markers 112 may be comprised of titanium or other similar metal.

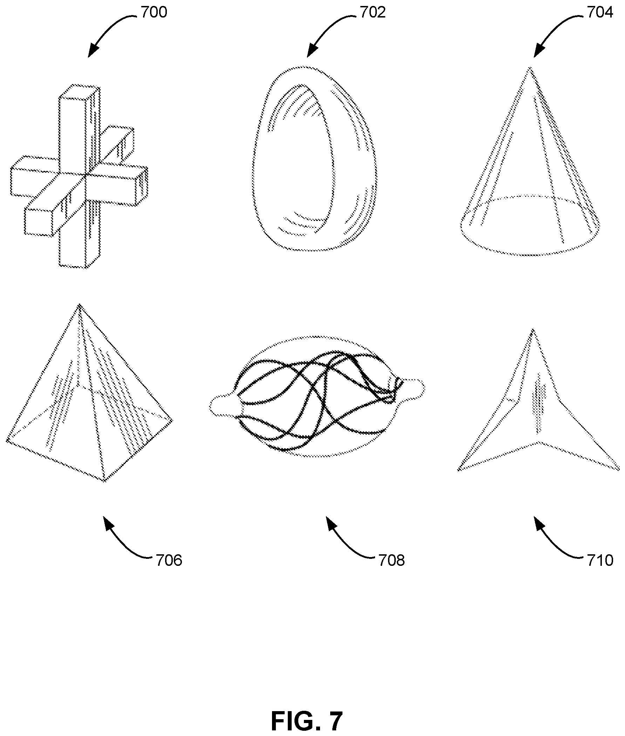

FIG. 7 depicts example frame configurations. The frame 108 may be formed in many different shapes and sizes. A size and shape of the frame 108, as well as structure, may be selected based at least in part on a size and a shape of the cavity 102. Additionally, in some examples, the size, shape, and structure selection for the frame 108 may be based on where growth of breast tissue initially occurs within the cavity 102.

Example shapes include a 3D cross 700, a 3D concave structure 702, a solid or hollow cone 704, a solid or hollow pyramid 706, a spherical or elliptical structure 708, and a 3D star 710. In some examples, the shape of the frame 108 may yield an open structure, such as the helix shape illustrated in FIG. 6. In other examples, the shape of the frame 108 may yield a partially open structure, such as the 3D concave structure 702, the hollow cone 704 or the hollow pyramid 706. In further examples, the shape of the frame 108 may yield a closed structure, such as the 3D cross 700, the solid cone 704, the solid pyramid 706, the spherical or elliptical structure 708, and the 3D star 710.

The example shapes provided in the figures are for illustrative purposes only, and are not intended to be limiting. An alternatively shaped frame 108 may be integrated with the hydrogel scaffold 106 to form the implantable device 100.

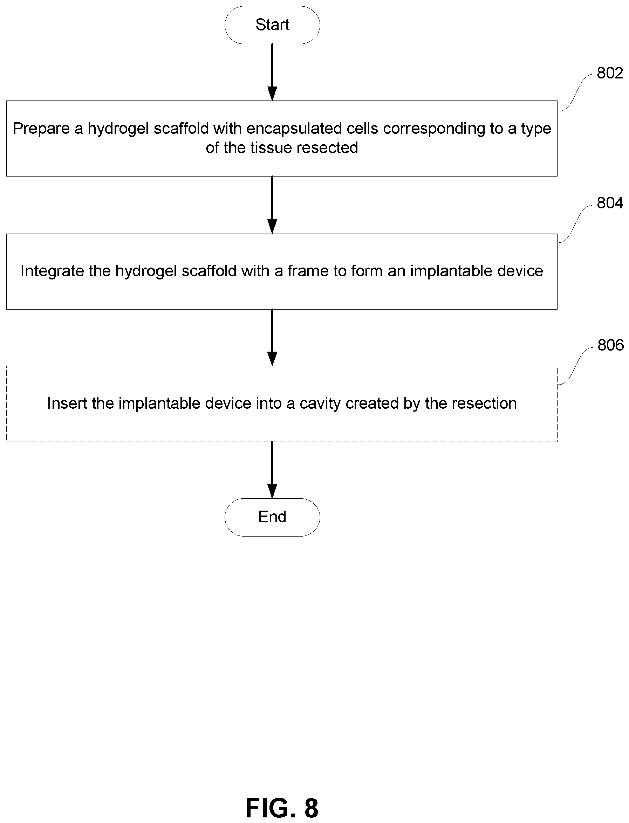

FIG. 8 depicts a method 800 to accelerate healing after a resection of tissue. At operation 802, a hydrogel scaffold 106 with encapsulated cells 110 corresponding to a type of the tissue resected is prepared. In some examples, the type of tissue resected is breast tissue. The cells 110 may be healthy cells of a patient 104 undergoing the resection. For example, the cells 110 may be obtained from healthy breast tissue of the patient 104 removed during a biopsy performed for diagnostic evaluation of the breast tissue (e.g., to determine whether the resection was necessary). The cells may be obtained by isolating tissue-specific cells from the healthy breast tissue, harvesting and expanding the cells in vitro, and mixing the cells with a hydrogel precursor solution to form a precursor-cell solution. The precursor-cell solution may be transitioned into a semi-solid material using physical crosslinking or chemical crosslinking (e.g. via gelation) to form the hydrogel scaffold 106 after the frame 108 has been integrated, as described in operation 804.

At operation 804, the hydrogel scaffold 106 may be integrated with a frame 108 to form an implantable device 100. A shape, size, and structure of the frame 108 may be selected based at least in part on a size and shape of a cavity 102 into which the implantable device 100 is to be inserted at optional operation 806. The frame 108 may be bioabsorbable and include one or more non-bioabsorbable, radiopaque markers 112 spaced along one or more portions of the frame 108. The one or more non-bioabsorbable, radiopaque markers can be made from any biocompatible, radio-opaque material (e.g., titanium, stainless steel, gold, or composite polymer materials) and may be attached to the portions of frame 108 using preformed holes in the frame into which the markers can be attached.

To integrate the hydrogel scaffold 106 with the frame 108, the precursor-cell solution may be applied to one or more portions of the frame 108, and gelation may be initiated. In some examples, the one or more portions of the frame 108 may be placed or positioned in the hydrogel precursor solution, which is contained within a receptacle, such as a petri dish or well plate, among other similar receptacles. Upon gelation, the one or more portions of the frame 108 may be at least partially surrounded by the hydrogel scaffold 106. To encapsulate the frame 108 in the hydrogel scaffold 106, the frame 108 may be submerged in the hydrogel precursor solution prior to gelation. In other examples, if the frame 108 has at least a partially open structure, the hydrogel precursor solution may be applied to the open structure, and gelation may be initiated to form the hydrogel scaffold 106. Upon gelation, the hydrogel scaffold 106 may be formed within the frame 108. In some examples, the integration of the hydrogel scaffold 106 and the frame 108 may be based on where growth of breast tissue initially occurs within the cavity 102.

At optional operation 806, the implantable device 100 may be inserted into a cavity 102 created by the resection. The bioabsorbable frame 108 may provide temporary structural support in the cavity 102 to reduce deformities in the look and appearance of the breast, while the encapsulated cells 110 within the bioabsorbable hydrogel scaffold 106 interact with native cells to facilitate new tissue formation within the hydrogel scaffold and other areas of the cavity. Additionally, the markers 112 may remain at a site of the resection for a prolonged period of time (e.g., remain after the hydrogel scaffold 106 and frame 108 have been absorbed) to enable identification of the site via imaging processes, which may be important for subsequent procedures and screenings.

In light of the foregoing, it should be appreciated that the present technology is able to accelerate healing post-resection using a hydrogel-based implantable device to facilitate tissue formation within a resection cavity. For example, the implantable device may include a hydrogel scaffold having healthy cells of the patient undergoing the resection encapsulated within that interact with native cells to facilitate new tissue formation within the hydrogel scaffold and other areas of the cavity. Additionally, the hydrogel scaffold may be integrated with a bioabsorbable frame to provide temporary structural support in the cavity while the tissue formation is being facilitated to reduce deformities in the look and appearance of the breast. The frame may also include non-bioabsorbable, radiopaque markers to enable identification of a site of the resection for subsequent procedures, screenings, or other imaging purposes.

The examples described herein may be employed using software, hardware, or a combination of software and hardware to implement and perform the systems and methods disclosed herein. Although specific devices have been recited throughout the disclosure as performing specific functions, one of skill in the art will appreciate that these devices are provided for illustrative purposes, and other devices may be employed to perform the functionality disclosed herein without departing from the scope of the disclosure.

Although specific examples are described herein, the scope of the technology is not limited to those specific examples. One skilled in the art will recognize other examples or improvements that are within the scope and spirit of the present technology. Therefore, the specific structure, acts, or media are disclosed only as illustrative examples. The scope of the technology is defined by the claims.

## Claims

1. A method for preparing an implantable device to accelerate healing after a resection of tissue, the method comprising:
preparing a hydrogel precursor solution;
forming a hydrogel scaffold with encapsulated cells corresponding to a type of the tissue resected by initiating gelation of the hydrogel precursor solution; and
integrating the hydrogel scaffold with a frame to form an implantable device for insertion into a cavity created by the resection;
wherein integrating the hydrogel scaffold with the frame comprises:
applying the hydrogel precursor solution to the one or more portions of the frame; and
initiating the gelation of the hydrogel precursor solution applied to the one or more portions of the frame to form the hydrogel scaffold that at least partially surrounds one or more portions of the frame.

2. The method of claim 1, wherein preparing the hydrogel scaffold with the encapsulated cells comprises:
removing a sample of tissue during a biopsy, wherein the sample of tissue is a same type of tissue as the tissue resected;
isolating cells from the sample of tissue;
harvesting and expanding the cells in vitro;
mixing the cells in the hydrogel precursor solution to form a precursor-cell solution; and
initiating gelation of the precursor-cell solution to form the hydrogel scaffold with the encapsulated cells.

3. The method of claim 2, wherein removing the sample of tissue during the biopsy comprises:
removing the sample of tissue during the biopsy of a patient undergoing the resection such that the cells encapsulated in the hydrogel scaffold are cells of the patient, wherein the sample of tissue removed comprises healthy tissue.

4. The method of claim 1, further comprising:
encapsulating the frame in the hydrogel scaffold by submerging the frame in the hydrogel precursor solution prior to the gelation.

5. The method of claim 1, further comprising:
applying the hydrogel precursor solution to an at least partially open structure of the frame such that the hydrogel scaffold is formed within the frame upon the gelation.

6. The method of claim 1, further comprising:
determining an application of the hydrogel precursor solution to the one or more portions of the frame based on growth patterns of new tissue formed in the cavity.

7. The method of claim 1, further comprising:
selecting at least one of a size, a shape, and a structure of the frame based at least in part on at least one of a size and a shape of the cavity.

8. An implantable device to accelerate healing after a resection of tissue, the implantable device comprising:
a frame comprising one or more portions; and
a hydrogel scaffold including encapsulated cells that correspond to a type of the tissue resected to facilitate tissue formation within a resection cavity upon insertion of the implantable device into the resection cavity, the hydrogel scaffold formed by initiation of gelation of a hydrogel precursor solution;
wherein one or more portions of the frame are at least partially surrounded by the hydrogel scaffold.

9. The implantable device of claim 8, wherein the frame is encapsulated in the hydrogel scaffold.

10. The implantable device of claim 8, wherein the frame has an at least partially open structure, and the hydrogel scaffold is formed within the frame.

11. The implantable device of claim 8, wherein the hydrogel scaffold and the frame are bioabsorbable.

12. The implantable device of claim 8, wherein the frame includes a plurality of markers positioned along the one or more portions of the frame.

13. The implantable device of claim 12, wherein the plurality of markers are comprised of a non-bioabsorbable, radiopaque material.

14. The implantable device of claim 8, wherein at least one of a size, a shape, and a structure of the frame is selected based at least in part on at least one of a size and a shape of the resection cavity.

## Patentansprüche

1. Verfahren zum Herstellen einer implantierbaren Vorrichtung zum Beschleunigen der Heilung nach einer Geweberesektion, wobei das Verfahren umfasst:
Herstellen einer Hydrogel-Vorläuferlösung;
Bilden eines Hydrogel-Gerüsts mit eingekapselten Zellen, die einem Typ des resezierten Gewebes entsprechen, indem die Gelierung der Hydrogel-Vorläuferlösung initiiert wird; und
Integrieren des Hydrogel-Gerüsts in einen Rahmen, um eine implantierbare Vorrichtung zum Einsetzen in einen durch die Resektion entstandenen Hohlraum zu bilden;
wobei das Integrieren des Hydrogel-Gerüsts in den Rahmen umfasst:
Auftragen der Hydrogel-Vorläuferlösung auf den einen oder die mehreren Abschnitte des Rahmens; und
Initiieren der Gelierung der Hydrogel-Vorläuferlösung, die auf den einen oder die mehreren Abschnitte des Rahmens aufgetragen wird, um das Hydrogel-Gerüst zu bilden, das einen oder mehrere Abschnitte des Rahmens mindestens teilweise umgibt.

2. Verfahren nach Anspruch 1, wobei das Herstellen des Hydrogel-Gerüsts mit den eingekapselten Zellen umfasst:
Entnehmen einer Gewebeprobe während einer Biopsie, wobei es sich bei der Gewebeprobe um denselben Gewebetyp handelt wie bei dem resezierten Gewebe;
Isolieren von Zellen aus der Gewebeprobe;
Ernten und Vermehren der Zellen in vitro;
Mischen der Zellen in der Hydrogel-Vorläuferlösung, um eine Vorläuferzelllösung zu bilden; und
Initiieren der Gelierung der Vorläuferzelllösung, um das Hydrogel-Gerüst mit den eingekapselten Zellen zu bilden.

3. Verfahren nach Anspruch 2, wobei das Entnehmen der Gewebeprobe während der Biopsie umfasst:
Entnehmen der Gewebeprobe während der Biopsie an einem Patienten, der sich der Resektion unterzieht, sodass die im Hydrogel-Gerüst eingekapselten Zellen Zellen des Patienten sind, wobei die entnommene Gewebeprobe gesundes Gewebe umfasst.

4. Verfahren nach Anspruch 1, ferner umfassend:
Einkapseln des Rahmens in das Hydrogel-Gerüst durch Eintauchen des Rahmens in die Hydrogel-Vorläuferlösung vor der Gelierung.

5. Verfahren nach Anspruch 1, ferner umfassend:
Auftragen der Hydrogel-Vorläuferlösung auf eine mindestens teilweise offene Struktur des Rahmens, so dass beim Gelieren das Hydrogel-Gerüst innerhalb des Rahmens gebildet wird.

6. Verfahren nach Anspruch 1, ferner umfassend:
Bestimmen eines Auftragens der Hydrogel-Vorläuferlösung auf den einen oder die mehreren Abschnitte des Rahmens basierend auf Wachstumsmustern von neuem Gewebe, das in dem Hohlraum gebildet wird.

7. Verfahren nach Anspruch 1, ferner umfassend:
Auswählen von mindestens einem von einer Größe, einer Form und einer Struktur des Rahmens mindestens teilweise basierend auf mindestens einem von einer Größe und einer Form des Hohlraums.

8. Implantierbare Vorrichtung zum Beschleunigen der Heilung nach einer Geweberesektion, wobei die implantierbare Vorrichtung umfasst:
einen Rahmen, der einen oder mehreren Abschnitte umfasst; und
ein Hydrogel-Gerüst, das eingekapselte Zellen einschließt, die einem Typ des resezierten Gewebes entsprechen, um die Gewebebildung innerhalb eines Resektionshohlraums beim Einsetzen der implantierbaren Vorrichtung in den Resektionshohlraum zu erleichtern, wobei das Hydrogel-Gerüst durch Initiieren der Gelierung einer Hydrogel-Vorläuferlösung gebildet wird;
wobei ein oder mehrere Abschnitte des Rahmens mindestens teilweise von dem Hydrogel-Gerüst umgeben sind.

9. Implantierbare Vorrichtung nach Anspruch 8, wobei der Rahmen in dem Hydrogel-Gerüst eingekapselt ist.

10. Implantierbare Vorrichtung nach Anspruch 8, wobei der Rahmen eine mindestens teilweise offene Struktur aufweist und das Hydrogel-Gerüst innerhalb des Rahmens gebildet ist.

11. Implantierbare Vorrichtung nach Anspruch 8, wobei das Hydrogel-Gerüst und der Rahmen biologisch absorbierbar sind.

12. Implantierbare Vorrichtung nach Anspruch 8, wobei der Rahmen eine Vielzahl von Markierungen einschließt, die entlang eines oder mehrerer Abschnitte des Rahmens positioniert sind.

13. Implantierbare Vorrichtung nach Anspruch 12, wobei die Vielzahl von Markierungen aus einem nicht biologisch absorbierbaren, röntgendichten Material besteht.

14. Implantierbare Vorrichtung nach Anspruch 8, wobei mindestens eines von einer Größe, einer Form und einer Struktur des Rahmens mindestens teilweise auf der Grundlage von mindestens einem von einer Größe und einer Form des Resektionshohlraums ausgewählt wird.

## Revendications

1. Procédé de préparation d'un dispositif implantable destiné à accélérer la cicatrisation après une résection de tissu, le procédé comprenant :
la préparation d'une solution de précurseur d'hydrogel ;
la formation d'un échafaudage d'hydrogel avec des cellules encapsulées correspondant à un type du tissu réséqué en initiant la gélification de la solution de précurseur d'hydrogel ; et
l'intégration de l'échafaudage d'hydrogel à un cadre afin de former un dispositif implantable à insérer dans une cavité créée par la résection ;
dans lequel l'intégration de l'échafaudage d'hydrogel au cadre comprend :
l'application de la solution de précurseur d'hydrogel sur la ou les parties du cadre ; et
l'initiation de la gélification de la solution de précurseur d'hydrogel appliquée à la ou aux parties du cadre afin de former l'échafaudage d'hydrogel qui entoure au moins partiellement une ou plusieurs parties du cadre.

2. Procédé selon la revendication 1, dans lequel la préparation de l'échafaudage d'hydrogel avec les cellules encapsulées comprend :
le prélèvement d'un échantillon de tissu au cours d'une biopsie, dans lequel l'échantillon de tissu est d'un même type de tissu que le tissu réséqué ;
l'isolement de cellules à partir de l'échantillon de tissu ;
la récolte et l'expansion des cellules in vitro ;
le mélange des cellules dans la solution de précurseur d'hydrogel afin de former une solution de cellules de précurseur ; et
l'initiation de la gélification de la solution de cellules de précurseur afin de former l'échafaudage d'hydrogel avec les cellules encapsulées.

3. Procédé selon la revendication 2, dans lequel le prélèvement de l'échantillon de tissu au cours de la biopsie comprend :
le prélèvement de l'échantillon de tissu au cours de la biopsie d'un patient soumis à la résection, de telle sorte que les cellules encapsulées dans l'échafaudage d'hydrogel sont des cellules du patient, dans lequel l'échantillon de tissu prélevé comprend du tissu sain.

4. Procédé selon la revendication 1, comprenant en outre :
l'encapsulation du cadre dans l'échafaudage d'hydrogel en submergeant le cadre dans la solution de précurseur d'hydrogel avant la gélification.

5. Procédé selon la revendication 1, comprenant en outre :
l'application de la solution de précurseur d'hydrogel à une structure au moins partiellement ouverte du cadre de telle sorte que l'échafaudage d'hydrogel est formé à l'intérieur du cadre lors de la gélification.

6. Procédé selon la revendication 1, comprenant en outre :
la détermination d'une application de la solution de précurseur d'hydrogel à la ou aux parties du cadre en fonction de schémas de croissance de nouveau tissu formé dans la cavité.

7. Procédé selon la revendication 1, comprenant en outre :
la sélection d'au moins l'une parmi une taille, une forme, et une structure du cadre en fonction au moins en partie d'au moins l'une parmi une taille et une forme de la cavité.

8. Dispositif implantable destiné à accélérer la cicatrisation après une résection de tissu, le dispositif implantable comprenant :
un cadre comprenant une ou plusieurs parties ; et
un échafaudage d'hydrogel comportant des cellules encapsulées qui correspondent à un type du tissu réséqué pour faciliter la formation de tissu à l'intérieur d'une cavité de résection lors de l'insertion du dispositif implantable dans la cavité de résection, l'échafaudage d'hydrogel étant formé par l'initiation de la gélification d'une solution de précurseur d'hydrogel ;
dans lequel une ou plusieurs parties du cadre sont au moins partiellement entourées par l'échafaudage d'hydrogel.

9. Dispositif implantable selon la revendication 8, dans lequel le cadre est encapsulé dans l'échafaudage d'hydrogel.

10. Dispositif implantable selon la revendication 8, dans lequel le cadre a une structure au moins partiellement ouverte, et l'échafaudage d'hydrogel est formé à l'intérieur du cadre.

11. Dispositif implantable selon la revendication 8, dans lequel l'échafaudage d'hydrogel et le cadre sont bioabsorbables.

12. Dispositif implantable selon la revendication 8, dans lequel le cadre comporte une pluralité de marqueurs positionnés le long de la ou des parties du cadre.

13. Dispositif implantable selon la revendication 12, dans lequel la pluralité de marqueurs est constituée d'un matériau radio-opaque non bioabsorbable.

14. Dispositif implantable selon la revendication 8, dans lequel au moins l'une parmi une taille, une forme, et une structure du cadre est choisie en fonction au moins en partie d'au moins l'une parmi une taille et une forme de la cavité de résection.
